# EUROPEAN PATENT SPECIFICATION

(11) **EP 0 984 919 B1**
(45) Date of publication and mention of the grant of the patent: **22.01.2003**
(21) Application number: 98911685.0
(22) Date of filing: 16.03.1998
(51) Int. Cl.: C07C 69/76, C07D 487/00, C07C 67/00, C07C 69/65

(54) **PROCESS FOR THE PREPARATION OF ARYLMALONATES**
VERFAHREN ZUR HERSTELLUNG VON ARYLMALONATEN
METHODE DE PREPARATION D'ARYLMALONATES

(30) Priority: 18.03.1997 US 820277; 18.03.1997 US 820268
(43) Date of publication of application: 15.03.2000
(73) Proprietor: BASF AKTIENGESELLSCHAFT, 67056 Ludwigshafen (DE)
(72) Inventor: KRUMMEL, Guenter, D-55578 Vendersheim (DE); KNELL, Marcus, D-55218 Ingelheim (DE)
(74) Representative: Köster, Reinhold, Dr.
(86) International application number: US9805125
(87) International publication number: WO98041496

(56) References cited:
- DE-A- 2 509 017
- JP-A- 53 037 637
- US-A- 2 367 632
- CHEMICAL ABSTRACTS, vol. 114, no. 17, 29 April 1991 (1991-04-29) Columbus, Ohio, US; abstract no. 163666h, SATYANARAYANA G. ET AL.: "Convenient one pot synthesis of esters of carboxylic acids from alkyl or aryl halides" page 721; column r; XP002137969 & SYNTH.COMMUN., vol. 20, no. 21, 1990, pages 3273-3276,

## Description

Arylmalonates are useful as intermediates for the preparation of a variety of compounds which are useful as agrochemicals, pharmaceuticals or liquid crystals. In particular, they are key intermediates in the preparation of fungicidal 6-aryltriazolopyrimidines.

Conventionally the preparation of these compounds is carried out in a 4-step synthesis starting from arylmethylhalides according to Scheme 1, below.

Scheme 1 Conventional process for the preparation of arylmalonates:

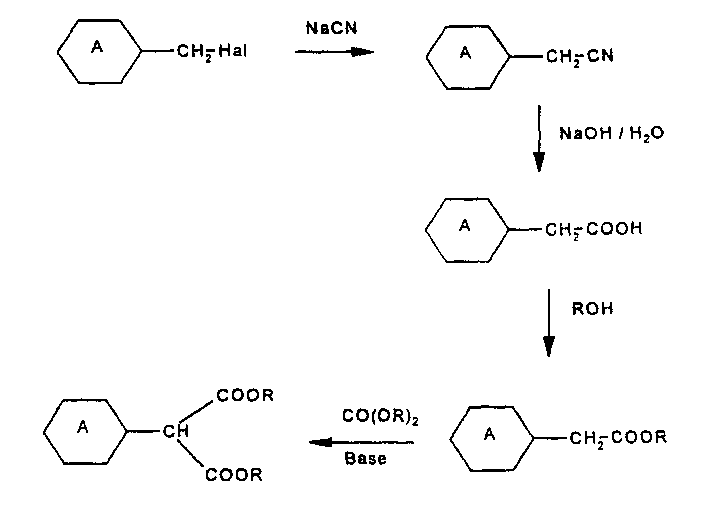

However, this method is not entirely satisfactory for large scale production, since highly toxic sodium cyanide is required and the overall yield of the reactions starting from arylmethylhalide is often low. Another process fear the preparation of Arymalonates is disclosed in US-A-2 367 632.

The present invention provides an effective and efficient process for the preparation of dialkyl arylmalonates of formula I, 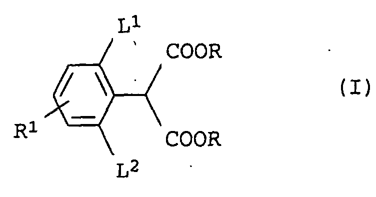 wherein R represents C₁-C₆ alkyl, L¹, L² each independently represent a halogen atom and R¹ represents a hydrogen or halogen atom or a C₁-C₆-alkyl or C₁-C₆ alkoxy group; which comprises treating an (a) arylmethylhalide of formula II 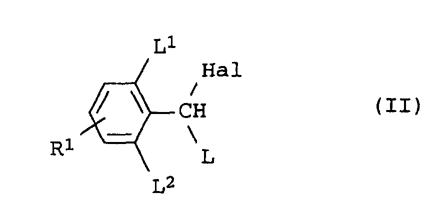 wherein Hal represents a halogen atom, preferably a chloro or bromo atom, and L represents a hydrogen with magnesium in an inert solvent, (b) the resuling Grignard reagent is treated with more than two moles of a dialkyl carbonate or an alkyl chloroformate, related to 1 mole of arylmethylhalide of formula II, and the resuling reaction mixture comprising an arylacetate of formula III 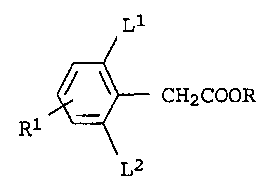 wherein L¹,L², R¹, and R have the meaning given, and the dialkyl carbonate or the alkyl chloroformate is treated with an alkali metal alkoxide.

It is, therefore, an object of the present invention to provide an efficient new process for the preparation of arylmalonates.

Other objects and advantages of the present invention will be apparent to those skilled in the art from the following description and the appended claims.

In general terms, unless otherwise stated, as used herein the term halogen atom may denote a bromine, iodine, chlorine or fluorine atom, and is especially a bromine or chlorine atom.

Optionally substituted moieties may be unsubstituted or have from one up to the maximal possible number of substituents. Typically, 0 to 2 substituents are present.

In general terms, unless otherwise stated herein, the term alkyl as used herein with respect to a radical or moiety refer to a straight or branched chain radical or moiety. As a rule, such radicals have up to 10, in particular up to 6 carbon atoms. Suitably an alkyl moiety has from 1 to 6 carbon atoms, preferably from 1 to 3 carbon atoms. A preferred alkyl moiety is a methyl or especially an ethyl group.

In general terms, unless otherwise stated herein, the term heteroaromatic group, as used herein with respect to a radical or moiety refers to a heteroaryl group having 6 ring atoms selected from carbon, nitrogen, oxygen and sulphur, at least one of which being nitrogen.

A preferred embodiment is

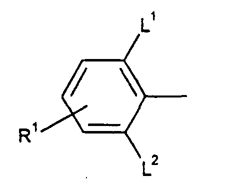

in which L¹ and L² each independently represent a halogen atom, preferably fluorine or chlorine, and

R¹ represent a hydrogen or halogen atom or an alkyl or alkoxy group.

In a particularly preferred embodiment ring A represents 2-chloro-6-fluorophenyl.

The invention preferably relates to a process for the preparation of dialkyl arylmalonates of formula I from an arylmethylhalide of formula II,
wherein L represents a hydrogen atom, which comprises the following steps:
(a) treating the arylmethylhalide of formula II with magnesium in an inert solvent,
(b) treating the resulting Grignard reagent with more than 2 moles of a dialkyl carbonate or an alkyl chloroformate related to 1 mole of arylmethylhalide of formula II, and
(c) treating the resulting reaction mixture comprising an arylacetate of formula III, 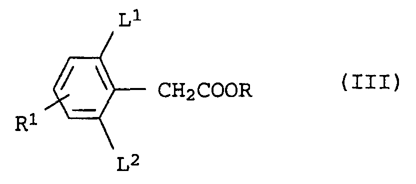
wherein R has the meaning given, and the dialkyl carbonate or the alkyl chloroformate with a base.

The process for the preparation of dialkyl arylmalonates of formula I which comprises treating an arylmethylhalide of formula II, wherein L represents a halogen atom, with at least two moles magnesium related to 1 mole of arylmethylhalide of formula II, in an inert solvent, and with at least two moles of a dialkyl carbonate or an alkyl chloroformate, in particular a dialkyl carbonate, related to 1 mole of arylmethyldihalide of formula II, does und fall within the scope of the present claims and represents a comparative

Further preferred embodiments of the process according to the present invention is a process wherein: the magnesium used is activated with 1,2-dibromoethane (DBE) or diethylether (DEE);
1 mole of arylmethylhalide of formula II, in which L represents Hal, is treated with 2.1 to 5.0, preferably 2.2 to 4.2 moles magnesium;
1 mole of arylmethylhalide of formula II, in which L represents a hydrogen atom, is treated with 1.1 to 3.5 moles magnesium, preferably 1.3 to 3.1 moles magnesium ;
a mixture comprising the arylmethylhalide of formula II, wherein L represents a halogen atom, the dialkyl carbonate and optionally an inert solvent is added, preferably slowly dosed, to a mixture comprising magnesium and an inert solvent;
the reaction is carried out in the presence of an inert solvent selected from the group consisting of diethylether (DEE), diisopropylether, *tert*-butylmethylether (TBME), dimethoxymethane (DMM), 2,2-dimethoxypropane (DMP), diethoxyethane, tetrahydrofuran (THF), tetrahydropyran (THP), toluene, mesitylene, glyme or a mixture of these solvents,
the reaction is carried out in the presence of a tertiary amine, in particular pyridine, tri-n-butylamine (TBA) or TMEDA;
the reaction is carried out at temperatures between 0 °C and 100 °C, preferably between 25 °C and 50 °C with arylmethylhalides of formula II, in which L represents a hydrogen atom, or between 50 °C and 95 °C with arylmethylhalides of formula II, in which L represents a halogen atom;
1 mole of arylmethylhalide of formula II is treated with 3 to 12 moles of the dialkyl carbonate or 3 to 12 moles of the alkyl chloroformate;
the reaction mixture obtained by reacting the compound of formula II with magnesium and the dialkyl carbonate or the alkyl chloroformate is treated with a base;
the reaction mixture obtained by reacting the compound of formula II,
wherein L represents a halogen atom, with magnesium and the dialkyl carbonate or the alkyl chloroformate is heated until the products have been completely dissolved and is transferred to a mixture comprising the base and the dialkyl carbonate or the alkyl chloroformate; the base is an alkali metal alkoxide; and the reaction mixture is heated up to 150 °C upon adding the base.

Further preferred embodiments of the process according to the present invention using the arylmethylhalides of formula II, wherein L represents a hydrogen atom, is a process wherein:
in step (b) the Grignard reagent is treated with 4 to 12 moles of the dialkyl carbonate, in particular diethyl carbonate (DEC), or with 4 to 12 moles of the alkyl chloroformate, in particular ethyl chloroformate (ECF), related to
1 mole of arylmethylhalide of formula II;
the reaction of step (b) is carried out at temperatures between - 80 °C and 0 °C, preferably at - 40 °C to - 5 °C;
the reaction mixture obtained in step (b) is treated with a diluted aqueous acid and the organic phase comprising the arylacetate of formula III and
the dialkyl carbonate or the alkyl chloroformate is separated from the aqueous layer;
the reaction of step (c) is carried out with 1.1 to 3.0 moles of the base related to 1 mole of arylmethylhalide of formula II; and
the reaction of step (c) is carried out at temperatures between 80 °C and 160 °C.

As a rule, the initiation of the Grignard formation requires a long time in solvents like THF or DMM, therefore activation with DEE or DBE yields more reproducible results. Preferably a mixture of the arylmethylhalide and a solvent is slowly added to a mixture of magnesium, a solvent and 0.005 to 0.02 mol of the activator related to 1 mole of the arylmethylhalide.

Since, as a rule, an excess of magnesium is used, the remaining magnesium can be used as an active bottom in the following reaction without further activation.

Preferably, the reaction is carried out in mixtures of solvents and/or activators, in particular in mixtures essentially consisting of:
THF or DMM and DBE or DEE, as a general statement 1000 parts of THF or DMM to 0.5 - 2.0 parts of DBE; or toluene, DEE and TBA.

During the reaction with the dialkylcarbonates it is favourable to avoid that an excess of the free Grignard reagent is present, in particular
wherein Ar is a halogenated phenyl group, in order to prevent ketone formation or a Wurtz-type side reaction according to reaction schemes 2 and 3:

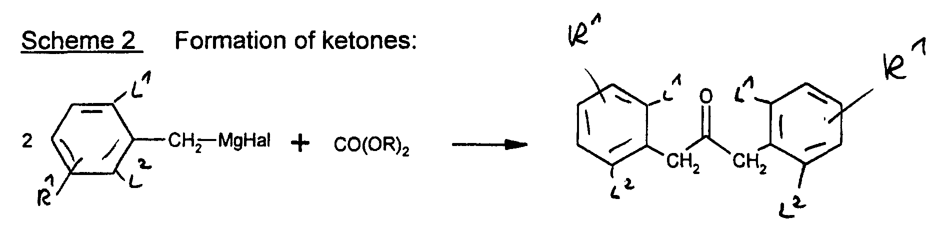

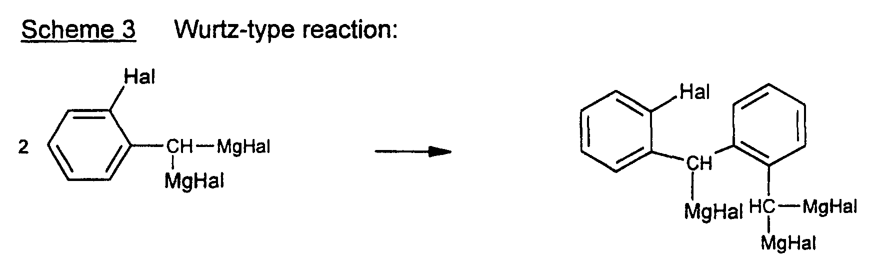

Therefore, the reaction mixture comprising the Grignard reagent is preferably added to the dialkyl carbonate or the alkyl chloroformate which are used in excess. Preferably, the Grignard reagent of the arylmethylhalide, wherein L represents a halogen atom, is generated insitu in the presence of the dialkyl carbonate which is used in excess. In a particularly preferred embodiment a mixture of the arylmethylhalide of formula II, wherein L represents a halogen atom, and the dialkyl carbonate, preferably diethyl carbonate, is added to a mixture of the magnesium, the inert solvent, in particular THF, and the activator, in particular DBE.

As a rule, the reaction between the Grignard reagent obtained from the arylmethylhalide of formula II, wherein L represents a hydrogen atom, and the dialkyl carbonate or the alkyl chloroformate is carried out at low temperatures, preferably below 0 °C, in particular between -10 °C and -25 °C.

As a rule, the reaction between the Grignard reagent obtained from the arylmethylhalide of formula II, wherein L represents a halogen atom, and the dialkyl carbonate or the alkyl chloroformate is carried out at elevated temperatures, preferably between 0 °C and 100 °C, in particular between 50 °C and 95 °C, most preferred between 60 °C and 85 °C.

In a particularly preferred embodiment of the invention the process using the arylmethylhalide of formula II, wherein L represents a hydrogen atom, as a starting material is carried out as follows:
- In principle, it is possible to treat the arylacetate of formula III formed in step (b) with the base in a one-pot synthesis, i.e. by adding the base to the reaction mixture. However, the inorganic salts present in this reaction mixture require the use of a high excess of the base in order to bind the halide ions present.
- Therefore, the reaction mixture obtained in step (b) preferably is neutralized with a diluted acid, in order to facilitate the separation of inorganic salts.
- Then the base is added to the separated organic layer, preferably after the solvent has been partly distilled off.
- Preferred bases are metal alkoxides, in particular sodium alkoxides as for example sodium methoxide or sodium ethoxide.
- After addition of the base, as a rule, the reaction mixture is heated and the alcohol formed from the dialkyl carboxylate is distilled off.

In a preferred embodiment, the process using the arylmethylhalide of formula II, wherein L represents a halogen atom, as starting material, is carried out as follows:
- In order to reduce the formation of an arylacetate of formula III as a side-product, the reaction is preferably carried out in the presence of a base, most preferred the reaction mixture obtained by the reaction of the Grignard reagent and the dialkyl carbonate or the alkyl chloroformate is treated with a base. However, the desired product can be obtained in high yields even without post-treatment with a base.
- The reaction mixture is preferably added to the base upon heating to temperatures above 100 °C. At this temperatures the reaction mixture becomes a colored clear solution and can be easily transferred to a solution which essentially consists of the base, optionally an inert solvent and/or a dialkyl carbonate.
- Preferred bases are metal alkoxides, in particular sodium alkoxides as for example sodium methoxide, sodium ethoxide or sodium *tert*-butoxide.
- After addition to the base the reaction mixture is , as a rule, heated up to 150 °C, preferably to 110 °C - 135 °C, most preferred to about 120 °C, and the alkanol formed from the dialkyl carboxylate is distilled off. The surplus dialkyl carbonate is preferably distilled off under reduced pressure.

The reaction is as a rule completed within 0.2 to 50, in particular 0.4 to 40 hours.

The remaining reaction mixture preferably is neutralized with diluted acid, the phases are separated and the organic layer is dried and concentrated.

The crude product obtained can be purified according to standard methods for example by distillation in vacuo, chromatographic methods or crystallization.

However, the crude product obtained according to the process of this invention is pure enough to be used as intermediate without further purification, in particular for the preparation of 6-aryltriazolopyrimidines of formula IV 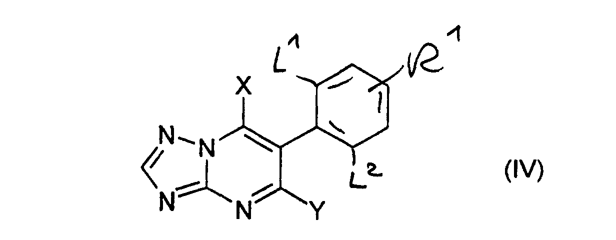 in which L¹, L² and R¹ have the meaning given for formula I, and
X represents a halogen atom, a hydroxy group or an optionally substituted amino group, and
Y represents a halogen atom or a hydroxy group.

The compounds of formula IV are prepared by the reaction of the dialkyl arylmalonates of formula I obtained according to the present invention with 3-amino-1,2,4-triazole as disclosed by EP 0 770 615. The resulting 5,7-dihydroxytriazolopyrimidine of formula IV, in which X and Y represent a hydroxy group, is halogenated and the resulting 5,7-dihalotriazolpyrimidine of formula IV, wherein X and Y represent a halogen atom, is treated with primary or secondary amine.

### Example 1

### Preparation of diethyl 2-chloro-6-fluorophenylmalonate

A mixture of magnesium (27 g), dimethoxymethane (40 ml) and dibromoethane (0.5 ml) is heated to 43 °C under stirring. After 5 minutes a mixture of 2-chloro-6-fluorobenzylchloride (4 g) and dimethoxymethane (16 ml) is added to initiate the reaction and stirred for 20 minutes. Within 2 hours a mixture of 2-chloro-6-fluorobenzylchloride (96 g) and dimethoxymethane (384 ml) is dosed to the reaction mixture under stirring at 42 to 44 °C. The mixture is kept under reflux with stirring for 1.5 hours. Upon cooling down to 30 °C the reaction mixture is dosed to diethylcarbonate (340 ml) at -8 to -14 °C within 0.5 hours. The reaction mixture is held at -10 °C for 48 hours. Upon warming up to 1 °C the reaction mixture is neutralized with a mixture of concentrated HCI (60ml) and water (150 ml). The phases are separated and the organic phase is transferred to another reactor.
The reaction mixture is concentrated by distillation. Sodium ethoxide (46 g) is added after cooling down to 75 °C. The reaction mixture is heated to 120 °C and the remaining dimethoxymethane and the formed ethanol is distilled off. Upon cooling down to 20 °C the reaction mixture is neutralized with a mixture of concentrated HCI (60ml) and water (150 ml). The phases are separated and the organic phase is dried with magnesium sulfate and concentrated in vacuo to yield the crude product (128 g / 80 % yield).

### Examples 2 to 15

### Preparation of diethyl 2-chloro-6-fluorophenylmalonate

Analogously to example 1 2-chloro-6-fluorobenzylchloride (CFBC) is treated with magnesium, a carbonylation reagent and sodium ethoxide as base in different solvents and at different temperatures.
The relative amounts of the reactants and solvents, the reaction temperature of the carbonylation step and yields are shown in the following table I:

**Table I**

| Examples 2 to 15 | | | | | | | | |
|---|---|---|---|---|---|---|---|---|
| Example | Initiator | Solvent | Mg/ CFBC | Cabonylation agent | Cabonyl-ation agent/ CFBC | NaOEt/ CFBC | temperature (°C) | Yield (%) |
| **2** | DEE | TBA | 5 | DEC | 5 | 1.8 | -10 | 31 |
| **3** | DBE | DMM | 2 | DEC | 5 | 1.5 | -10 | 75 |
| **4** | DEE | MTBE | 2 | DEC | 5 | 1.8 | -10 | 75 |
| **5** | DEE | TBA/ TOL | 2 | DEC | 5 | 2.4 | -10 | 53 |
| **6** | DBE | DMM | 2 | ECF | 5 | 2.4 | -10 | 66 |
| **7** | DBE | DMM | 2 | ECF | 5 | 1.2 | 20 | 48 |
| **8** | DBE | DMM | 3 | DEC | 10 | 2.5 | -28 | 91 |
| **9** | DBE | DMM | 3 | DEC | 10 | 2.5 | -25 | 90 |
| **10** | DBE | DMM | 2 | DEC | 10 | 2 | -25 | 61 |
| **11** | DBE | DMM | 2.5 | DEC | 5 | 1.2 | -10 | 80 |
| **12** | DEE | DMM | 3 | DEC | 6 | 2.5 | -5 | 80 |
| **13** | DBE | DEE | 2 | DEC | 11 | 1.6 | -25 | 67 |
| **14** | DBE | DMM/ TBA | 2.5 | DEC | 5 | 1.6 | -10 | 64 |
| 15 | DEE | MES | 2 | DEC | 5 | 2.5 | -10 | 81 |

### Example 16

### Preparation of diethyl 2-chloro-6-fluorophenylmalonate

A mixture of magnesium (3.4 g), THF (5 ml) and dibromoethane (0.1 ml) is heated to 75 °C under stirring. After 5 minutes a mixture of 2-chloro-6-fluorobenzaldichloride (10 g), diethylcarbonate (45 ml) and THF (20 ml) is dosed over a period of 1 hours. The reaction mixture is heated to 75 to 80 °C and stirred for 1 hour. The reaction mixture is separated off from un-reacted magnesium. The solvent is distilled off under reduced pressure.
Upon diluting with toluene (50 ml) and cooling down to 20 °C the reaction mixture is neutralized with a mixture of concentrated HCI (250 ml) and water (40 ml). The phases are separated and the organic phase is dried with magnesium sulfate and concentrated in vacuo to yield the crude product (70 % yield), which is purified by distillation under reduced pressure. The obtained product shows the following properties:
bp: 115 °C at 0.1 mbar
¹H-NMR (DMSO, 300 MHz):
   δ (ppm) = 7.45 (m, 2H), 7.30 (m, 1H), 5.22 (s, 1H), 4.19 (q, 4H), 1.18 (t, 6H)

### Examples 17 to 25

### Preparation of dialkyl 2-chloro-6-fluorophenylmalonate

Analogously to example 16 2-chloro-6-fluorobenzaldichloride (BAC) is treated with magnesium, a carbonylation reagent in different solvents and at different temperatures.

The relative amounts of the reactants and solvents, the reaction temperature of the carbonylation step and yields are shown in table II in which the following abbreviations have been used:
- DBE: 1,2-dibromothane
- THF: tetrahydrofuran
- DEC: diethylcarbonate
- DMM: dimethoxymethane
- THP: tetrahydropyran
- DEE: diethylether
- DMP: 2,2-dimethoxypropane
- DIP: diisopropylether
- TBME: *tert*-butylmethylether
- TBA: tri-n-butylamine
- DMC: dimethylcarbonate

**Table II**

| Examples 17 to 25 | | | | | | | | |
|---|---|---|---|---|---|---|---|---|
| Example | Initiator | Solvent | Mg/ BAG | Cabonylation agent | Cabonylation agent / BAG | temperature (°C) | Time (hrs) | Yield (%) |
| **17** | DBE | THF | 3 | DEC | 10 | 65-85 | 0.5 | 61 |
| **18** | DBE | THP | 3 | DEC | 8 | 80 | 54 | 64 |
| **19** | DBE | DMM | 3 | DEC | 6 | 55 | 16 | 55 |
| **20** | DBE/ DEE | DMP | 3 | DEC | 4 | 85 | 60 | 26 |
| **21** | DBE/ DEE | DIP | 4 | DEC | 4 | 75 | 42 | 65 |
| **22** | DBE/ DEE | TBME | 4 | DEC | 4 | 70 | 42 | 60 |
| **23** | DBE/ DEE | pyridine | 3 | DEC | 4 | 65 | 20 | 17 |
| **24** | DBE | THF/ TBA | 3 | DEC | 2 | 85 | 1 | 22 |
| **25** | DBE | THF | 3 | DMC | 10 | 60-62 | 0.5 | 32 |

### Example 26

### Preparation of diethyl 2-chloro-6-fluorophenylmalonate

A mixture of magnesium (17 g), THF (75 ml) and dibromoethane (0.5 ml) is heated to 75 °C under stirring. After 5 minutes a mixture of 2-chloro-6-fluorobenzaldichloride (50 g) and diethylcarbonate (280 ml) is dosed over a period of 2 hours. The reaction mixture is heated to 75 to 80 °C and stirred for 1 hour. Upon heating to 100 °C the reaction mixture is transferred to a mixture of sodium ethoxide (6.4 g) and diethylcarboxylate (90 ml). The resulting mixture is heated to 125 °C and stirred for 12 hours. Further sodium ethoxide (38.4 g) is added to the reaction mixture. The surplus diethylcarbonate is distilled of under reduced pressure (105 °C, 250 mbar).
Upon diluting with toluene (200 ml) and cooling down to 20 °C the reaction mixture is neutralized with a mixture of concentrated HCI (110 ml) and water (200 ml). The phases are separated and the organic phase is dried with magnesium sulfate and concentrated in vacuo to yield the crude product (61 g / 81 % yield).

### Use Example

### Preparation of 5,7-dichloro-6-(2-chloro-6-fluorophenyl)-1,2,4-triazolo[1,5a]pyrimidine

A mixture of 3-amino-1,2,4-triazole (0.15 mol), diethyl 2-chloro-6-fluorophenylmalonate (0.15 mol, obtained from Example 26) and tributylamine (0.15 mole) is heated at 170 °C and ethanol formed during the reaction is distilled off. Subsequently, the reaction mixture is cooled to 130 °C and phosphorous oxychloride (0.45 mol) is added within 30 minutes. The reaction mixture is heated with reflux for 6 hours. A mixture of water and toluene (1.5 I, 6 : 5) is added slowly.The organic phase is separated, washed with dilute hydrochloric acid and water, dried an concentrated in vacuo to yield a brown viscous oil (45 g) which contains 85 % of the title product. The title product is reacted without further purification with 4-methylpiperidine, which reaction is described in the prior art, to yield fungicidal 5-chloro-6-(2-chloro-6-fluorophenyl)-7-(4-methylpiperid-1-yl)-1,2,4-triazolo[1,5a]pyrimidine.

## Claims

1. A process for the preparation of dialkyl arylmalonates of formula I, 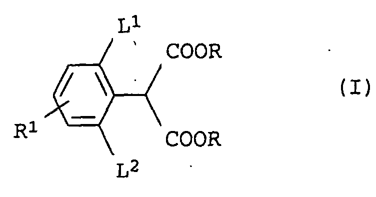 wherein R represents C₁-C₆-alkyl, L¹ and L² each independently represent a halogen atom and R¹ represent a hydrogen or halogen atom or a C₁-C₆-alkyl or C₁-C₆-alkoxy group;
**characterized in that**
(a) an arylmethylhalide of formula II, 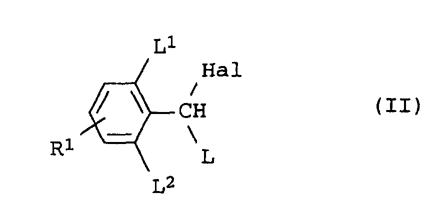 wherein L¹, L² and R¹ have the meaning given for formula I,
Hal represents a halogen atom, and
L represents a hydrogen atom,
is treated with magnesium in an inert solvent,
(b) the resulting Grignard reagent is treated with more than 2 moles of a dialkyl carbonate or an alkyl chloroformate related to 1 mole of arylmethylhalide of formula II, and
(c) the resulting reaction mixture comprising an arylacetate of formula III, 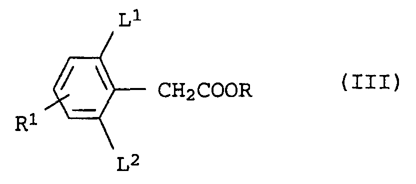
wherein L¹, L², R¹ and R have the meaning given, and the dialkyl carbonate or the alkyl chloroformate is treated with an alkali metal alkoxide.

2. A process according to Claim 1 wherein the reaction of step (a) is carried out at temperatures between 0 and 100°C.

3. A process according to any one of Claims 1 or 2, wherein the reaction of step (b) is carried out at temperatures between -80°C and 0°C.

4. A process according to any one of Claims 1 or 3, wherein the reaction mixture obtained in step (b) is treated with a diluted aqueous acid and the organic phase comprising the arylacetate of formula III and the dialkyl carbonate or the alkyl chloroformate is separated from the aqueous layer.

5. A process according to any of the Claims 1 to 4 wherein the reaction of step (c) is carried out with 1.1 to 3.0 moles of the base related to 1 mole of arylmethylhalide of formula II.

6. A process according to any of the Claims 1 to 5, wherein the reaction of step (c) is carried out at temperatures between 80°C and 160°C.

7. A process according to any one of Claims 1 to 6 wherein 1 mole of arylmethylhalide of formula II, is treated with 1.1 to 3.5 moles magnesium.

8. A process according to any of the Claims 1 to 7 wherein the reaction mixture is heated up to 150°C upon adding the base.

9. A process according to any of the preceding claims, wherein the magnesium used is activated with 1,2-dibromoethane and/or diethylether.

10. A process according to any of the preceding claims, wherein the inert solvent if selected from the group consisting of diethylether, diisopropylether, tert-butyl-methylether, dimethoxymethane, 2,2-dimethoxypropane, diethoxyethane, tetrahydrofuran, tetrahydropyran, toluene, glyme, pyridine, TMEDA and mesitylene, or a mixture of these solvents.

11. A process according to any of the preceding claims, wherein 1 mole of arylmethylhalide of formula II is treated with 3 to 12 moles of the dialkyl carbonate or 3 to 12 moles of the alkyl chloroformate.

12. A process for the preparation of a 6-phenyltriazolopyrimidine of formula IV, 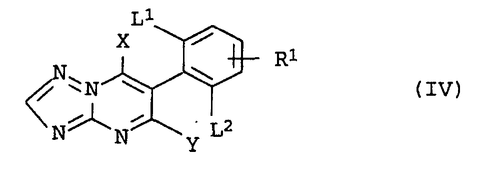 in which L¹, L² and R¹ have the meaning given for formula I,
X represents an optionally substituted amino group, and
Y represents a halogen atom, which comprises the steps of
(i) reacting of a dialkyl arylmalonate of formula I with 3-amino-1,2,4-triazole,
(ii) halogenating the resulting 5,7-dihydroxy-6-aryltriazolopyrimidine, and
(iii) treating the resulting 5,7-dihalotriazol-6-arylpyrimidine with primary or secondary amine,
**characterized in that** the dialkyl arylmalonate of formula I is prepared according to the process as claimed in any of the preceding claims.

## Patentansprüche

1. Verfahren zur Herstellung von Arylmalonsäuredialkylestern der Formel I 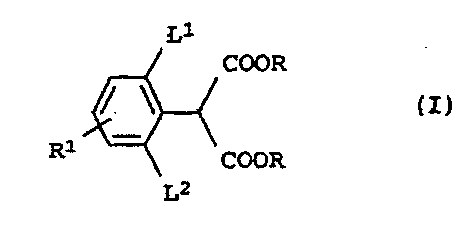 wobei R für C₁-C₆-Alkyl steht, L¹ und L² jeweils unabhängig voneinander für ein Halogenatom stehen und R¹ für ein Wasserstoff- oder Halogenatom oder eine C₁-C₆-Alkyl- oder C₁-C₆-Alkoxygruppe steht;
**dadurch gekennzeichnet, daß**
(a) man ein Arylmethylhalogenid der Formel II 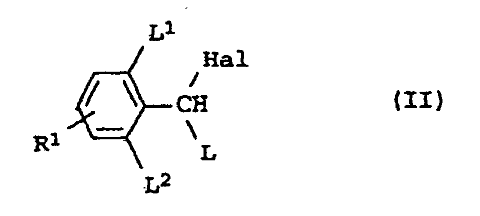 wobei L¹, L² und R¹ die für Formel I angegebene Bedeutung haben, Hal für ein Halogenatom steht und L für ein Wasserstoffatom steht, mit Magnesium in einem inerten Lösungsmittel behandelt,
(b) man die so erhaltene Grignard-Verbindung mit mehr als 2 mol eines Carbonsäuredialkylesters oder eines Chlorameisensäurealkylesters, bezogen auf 1 mol des Arylmethylhalogenids der Formel II, behandelt und
(c) man die so erhaltene Reaktionsmischung, die einen Arylessigsäureester der Formel III 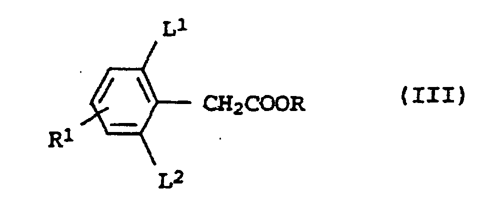
wobei L¹, L², R¹ und R die angegebene Bedeutung haben, und den Carbonsäuredialkylester oder den Chlorameisensäurealkylester enthält, mit einem Alkalialkoholat behandelt.

2. Verfahren nach Anspruch 1, bei dem man die Umsetzung von Schritt (a) bei Temperaturen zwischen 0 und 100°C durchführt.

3. Verfahren nach Anspruch 1 oder 2, bei dem man die Umsetzung von Schritt (b) bei Temperaturen zwischen -80°C und 0°C durchführt.

4. Verfahren nach einem der Ansprüche 1 bis 3, bei dem man die in Schritt (b) erhaltene Reaktionsmischung mit einer verdünnten wäßrigen Säure behandelt und die den Arylessigsäureester der Formel III und den Kohlensäuredialkylester oder den Chlorameisensäurealkylester enthaltende organische Phase von der wäßrigen Schicht abtrennt.

5. Verfahren nach einem der Ansprüche 1 bis 4, bei dem man die Umsetzung von Schritt (c) mit 1,1 bis 3,0 mol der Base, bezogen auf 1 mol des Arylmethylhalogenids der Formel II, durchführt.

6. Verfahren nach einem der Ansprüche 1 bis 5, bei dem man die Umsetzung von Schritt (c) bei Temperaturen zwischen 80°C und 160°C durchführt.

7. Verfahren nach einem der Ansprüche 1 bis 6, bei dem man 1 mol Arylmethylhalogenid der Formel II mit 1,1 bis 3,5 mol Magnesium behandelt.

8. Verfahren nach einem der Ansprüche 1 bis 7, bei dem man die Reaktionsmischung beim Zusatz der Base auf bis zu 150°C erhitzt.

9. Verfahren nach einem der vorhergehenden Ansprüche, bei dem man das verwendete Magnesium mit 1,2-Dibromethan und/oder Diethylether aktiviert.

10. Verfahren nach einem der vorhergehenden Ansprüche, bei denen das inerte Lösungsmittel aus der aus Diethylether, Diisopropylether, tert.-Butylmethylether, Dimethoxymethan, 2,2-Dimethoxypropan, Diethoxyethan, Tetrahydrofuran, Tetrahydropyran, Toluol, 1,2-Dimethoxyethan, Pyridin, TMEDA und Mesitylen oder einer Mischung dieser Lösungsmittel bestehenden Gruppe ausgewählt ist.

11. Verfahren nach einem der vorhergehenden Ansprüche, bei dem man 1 mol Arylmethylhalogenid der Formel II mit 3 bis 12 mol des Carbonsäuredialkylesters oder 3 bis 12 mol des Chlorameisensäurealkylesters behandelt.

12. Verfahren zur Herstellung von 6-Phenyltriazolopyrimidin der Formel IV 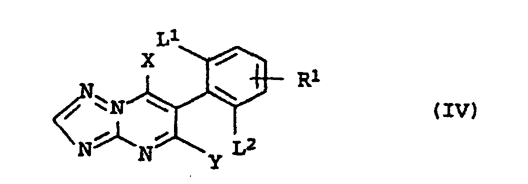 in welcher L¹, L² und R¹ die für Formel I angegebene Bedeutung haben,
X für eine gegebenenfalls substituierte Aminogruppe steht und
Y für ein Halogenatom steht, welches die folgenden Schritte umfaßt:
(i) Umsetzung eines Arylmalonsäuredialkylesters der Formel I mit 3-Amino-1,2,4-triazol,
(ii) Halogenierung des so erhaltenen 5,7-Dihydroxy-6-aryltriazolopyrimidins und
(iii) Behandeln des so erhaltenen 5,7-Dihalogentriazol-6-arylpyrimidins mit einem primären oder sekundären Amin,
**dadurch gekennzeichnet, daß** man den Arylmalonsäuredialkylester der Formel I durch das in einem der vorhergehenden Ansprüche beanspruchte Verfahren herstellt.

## Revendications

1. Procédé pour la préparation d'arylmalonates de dialkyle de formule I, 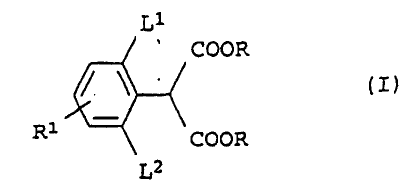 dans laquelle R représente un groupe alkyle en C₁-C₆, L¹ et L² représentent chacun indépendamment un atome d'halogène et R¹ représente un atome d'hydrogène ou d'halogène ou un groupe alkyle en C₁-C₆ ou alcoxy en C₁-C₆ ;
**caractérisé en ce que**
(a) un halogénure d'arylméthyle de formule II, 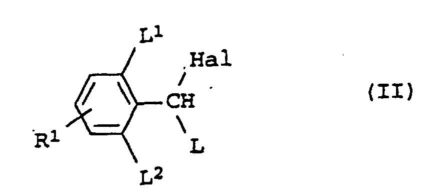 dans laquelle
L¹, L² et R¹ sont tels que définis pour la formule I,
Hal représente un atome d'halogène, et
L représente un atome d'hydrogène, est traité avec du magnésium dans un solvant inerte,
(b) le réactif de Grignard résultant est traité avec plus de 2 moles d'un carbonate de dialkyle ou d'un chloroformiate d'alkyle pour 1 mole d'halogénure d'arylméthyle de formule II, et
(c) le mélange réactionnel résultant comprenant un arylacétate de formule III, 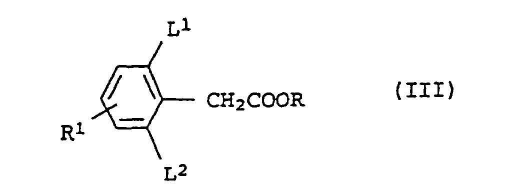
dans laquelle L¹, L², R¹ et R sont tels que définis, et le carbonate de dialkyle ou le chloroformiate d'alkyle est traité avec un alcoolate de métal alcalin.

2. Procédé selon la revendication 1, dans lequel la réaction de l'étape (a) est conduite à des températures comprises entre 0 et 100°C.

3. Procédé selon l'une quelconque des revendications 1 ou 2, dans lequel la réaction de l'étape (b) est conduite à des températures comprises entre -80°C et 0°C.

4. Procédé selon l'une quelconque des revendications 1 ou 3, dans lequel le mélange réactionnel obtenu dans l'étape (b) est traité avec un acide aqueux dilué et la phase organique comprenant l'arylacétate de formule III et le carbonate de dialkyle ou le chloroformiate d'alkyle est séparée de la phase aqueuse.

5. Procédé selon l'une quelconque des revendications 1 à 4, dans lequel la réaction de l'étape (c) est effectuée avec 1,1 à 3,0 moles de la base pour 1 mole de l'halogénure d'arylméthyle de formule II.

6. Procédé selon l'une quelconque des revendications 1 à 5, dans lequel la réaction de l'étape (c) est conduite à des températures comprises entre 80°C et 160°C.

7. Procédé selon l'une quelconque des revendications 1 à 6, dans lequel 1 mole d'halogénure d'arylméthyle de formule II est traitée avec 1,1 à 3,5 moles de magnésium.

8. Procédé selon l'une quelconque des revendications 1 à 7, dans lequel le mélange réactionnel est chauffé à une température d'au plus 150°C lors de l'addition de la base.

9. Procédé selon l'une quelconque des revendications précédentes, dans lequel le magnésium utilisé est activé par le 1,2-dibromoéthane et/ou l'éther de diéthyle.

10. Procédé selon l'une quelconque des revendications précédentes, dans lequel le solvant inerte est choisi dans la classe formée par l'éther de diéthyle, l'éther de diisopropyle, l'éther de *tert*-butyle et de méthyle, le diméthoxyméthane, le 2,2-diméthoxypropane, le diéthoxyéthane, le tétrahydrofuranne, le tétrahydropyranne, le toluène, le glyme, la pyridine, TMEDA et le mésitylène, ou un mélange de ces solvants.

11. Procédé selon l'une quelconque des revendications précédentes, dans lequel 1 mole d'halogénure d'arylméthyle de formule II est traitée avec 3 à 12 moles du carbonate de dialkyle ou 3 à 12 moles du chloroformiate d'alkyle.

12. Procédé pour la préparation d'une 6-phényltriazolopyrimidine de formule IV, 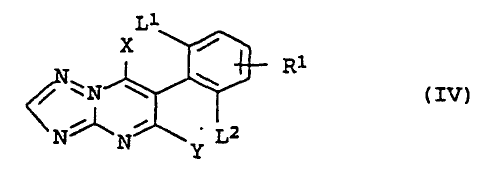 dans laquelle
L¹, L² et R¹ sont tels que définis pour la formule I,
X représente un groupe amino facultativement substitué, et
Y représente un atome d'halogène, qui comprend les étapes suivantes
(i) faire réagir un arylmalonate de dialkyle de formule I avec le 3-amino-1,2,4-triazole,
(ii) halogéner la 5,7-dihydroxy-6-aryltriazolopyrimidine résultante, et
(iii) traiter la 5,7-dihalogénotriazole-6-arylpyrimidine
résultante avec une amine primaire ou secondaire,
**caractérisé en ce que** l'arylmalonate de dialkyle de formule I est préparé selon le procédé revendiqué dans l'une quelconque des revendications précédentes.
